# EUROPÄISCHE PATENTSCHRIFT

(11) **EP 0 153 786 B1**
(45) Veröffentlichungstag und Bekanntmachung des Hinweises auf die Patenterteilung: **13.01.1993**
(21) Anmeldenummer: 85200216.1
(22) Anmeldetag: 20.02.1985
(51) Int. Cl.: G01N 23/04, G01T 1/29, G01N 23/201, A61B 6/00

(54) **Röntgengerät**
X-ray apparatus
Appareil à rayons X

(30) Priorität: 25.02.1984 DE 3406905
(43) Veröffentlichungstag der Anmeldung: 04.09.1985
(73) Patentinhaber: Philips Patentverwaltung GmbH, 22335 Hamburg (DE); Philips Electronics N.V., 5621 BA Eindhoven (NL)
(72) Erfinder: Harding, Geoffrey, Dr., D-2000 Hamburg 52 (DE); Kosanetzky, Josef-Maria, Dr., D-2000 Norderstedt 1 (DE); Neitzel, Ulrich, Dr., D-2000 Hamburg 65 (DE); Ypma, Peter, NL-5621 BA Eindhoven (NL)
(74) Vertreter: Hartmann, Heinrich, Dipl.-Ing.

(56) Entgegenhaltungen:
- EP-A- 0 028 431
- DE-A- 2 639 631
- FR-A- 2 249 517
- GB-A- 2 054 319
- GB-A- 2 083 969
- US-A- 3 927 318
- US-A- 3 936 638
- US-A- 4 229 651
- MED. PHYS. 10(6), Nov/Dez 1983, Seiten 819-823

## Beschreibung

Die Erfindung betrifft ein Röntgengerät nach dem Oberbegriff des Anspruchs 1. Ein solches Gerät ist aus Med. Phys. 10(6), November/Dezember 1983, Seiten 819 bis 823, bekannt. Die Detektoranordnung besteht dabei aus einem einzigen Detektor, und zur Erfassung der unter unterschiedlichen Streuwinkeln gestreuten Strahlung wird der Detektor um den Durchstoßpunkt des Primärstrahls durch ein Testphantom herum geschwenkt. Dabei wird die Tatsache ausgenutzt, daß Streustrahlung, die mit der Richtung des Primärstrahls nur einen kleinen Winkel einschließt (z.B. kleiner als 12^{o}) überwiegend aus elastisch gestreuter Strahlung besteht. Im Gegensatz zu nicht elastisch gestreuter (Compton-Strahlung) entspricht das Energiespektrum elastisch gestreuter Strahlung dem des Primärstrahlenbündels. Die elastische Streustrahlung weist darüberhinaus eine sehr starke Winkelabhängigkeit auf mit einem ausgeprägten Maximumm, das abhängig von der bestrahlten Materie und von der Härte der Streustrahlung zwischen 1^{o} und 12^{o} liegt.

Ausgehend von der Erkenntnis, daß elastisch gestreute Strahlung in erheblichem Maße Information auch über den chemischen Zustand der durchstrahlten Materie enthält, liegt der Erfindung die Aufgabe zugrunde, ein Röntgengerät der eingangs genannten Art so auszugestalten, daß die elastische Streustrahlung und deren Winkelabhängigkeit erfaßt und zur Informationsgewinnung benutzt werden kann. Diese Aufgabe wird erfindungsgemäß durch die im kennzeichnenden Teil des Anspruchs 1 angegebenen Maßnahmen gelöst.

Da die elastische Streustrahlung in einem relativ kleinen Winkelbereich bezogen auf den primärstrahl auftritt, durchsetzt sie näherungsweise den gleichen Teil des Untersuchungsbereichs wie der Primärstrahl. Es kann daher davon ausgegangen werden, daß sie im gleichen Maße durch einen im Untersuchungsbereich befindlichen Körper geschwächt wird wie der Primärstrahl, so daß es relativ leicht ist, den Einfluß der Schwächung der Streustrahlung durch den Körper durch eine Korrektur zu eliminieren. Die auf diese Weise verarbeiteten und nach Streuwinkel getrennt gespeicherten Streusignale können auf verschiedene Weise zur Informationsgewinnung benutzt werden.

Beispielsweise ist es möglich, ein (zweidimensionales) Projektionsbild der gemessenen Integrale über die Streuverteilung längs des Strahlenweges für einen festen Streuwinkel auszugeben. Ferner kann aus einer Vielzahl von gemessenen Streuintegralen die Streudichteverteilung in einer Schicht rekonstruiert werden. Beide Darstellungen sind für verschiedene Streuwinkel möglich. Es ist aber auch möglich, in einem Bild, das die Absorptionsverteilung zweidimensional darstellt, einen oder mehrere Bildbereiche zu markieren und dafür (mittlere) Streudichte als Funktion des Streuwinkels auszugeben.

Die Erfindung ist bei allen Röntgenaufnahmeverfahren anwendbar, bei denen das Primärstrahlenbündel senkrecht zu seiner Ausbreitungsrichtung lediglich eine kleine Ausdehnung hat (pencil beam), und zwar auch dann, wenn mehrere derartige Primärstrahlen benutzt werden.

Eine Ausgestaltung der Erfindung ist in Anspruch 2 beschrieben.

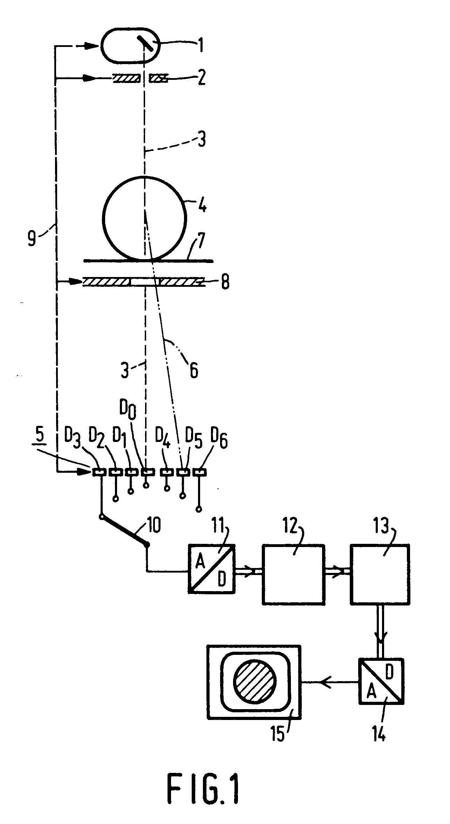

Die Erfindung wird nachstehend anhand der Zeichnung näher erläutert. Es zeigt Fig. 1 eine Ausführungsform.

In Fig. 1 ist mit 1 eine Strahlenquelle in Form eines Röntgenstrahlers bezeichnet, in deren Strahlengang eine Blende 2 angeordnet ist, die einen Primärstrahl 3 mit geringem Querschnitt senkrecht zu seiner Ausbreitungsrichtung (pencil beam) ausblendet, der einen Untersuchungsbereich mit einem Körper 4, der ein Patient jedoch auch ein technisches Objekt sein kann, durchsetzt. Jenseits des Körpers 4 - in bezug auf die Strahlenquelle - ist eine Detektoranordnung 5 angeordnet, die aus einer Anzahl von Detektoren D₀...D₆ besteht, von denen der eine (D₀) genau im Primärstrahl 3 angeordnet ist, während die anderen Detektoren D₁, D₆ sich in einer zum Primärstrahl senkrechten Ebene bzw. auf einer Geraden in dieser Ebene befinden und vom Primärstrahl 3 unterschiedliche Abstände aufweisen. Die Detektoren D₀...D₆ haben solche Abmessungen und sind so weit vom Körper 4 entfernt angeordnet, daß jeder Detektor nur die Streustrahlen empfangen kann, die unter einem ganz bestimmten, dem jeweiligen Detektor zugeordneten Winkel aus dem Körper 4 austreten. Zur Veranschaulichung ist in Fig. 1 lediglich ein streustrahl 6 angedeutet, der vom Detektor D₅ erfaßt wird.

Zwischen dem Körper 4, der sich auf der Tischplatte 7 eines nicht näher dargestellten Untersuchungstisches befindet, und der Detektoranordnung 5 befindet sich eine weitere Blende 8, deren Öffnung so bemessen ist, daß eine Verbindungsgerade zwischen dem Primärstrahl 3 innerhalb des Körpers 4 und den äußeren Detektoren D₃ und D₆ gerade noch die Öffnung passieren kann. Sie unterdrückt jedoch mehrfach gestreute Strahlung. Wird beispielsweise ein Punkt auf der äußeren rechten Seite des Körpers 4 von (Compton-) Streustrahlung getroffen und emittiert dieser Punkt seinerseits wiederum Streustrahlung, dann werden zumindest die Detektoren D₄, D₅ und D₆ dagegen abgeschirmt. Noch besser läßt sich die Mehrfach-Streustrahlung unterdrücken, wenn zwischen der Detektoranordnung 5 und dem Körper 4 Lamellen so angeordnet sind, daß jeder Detektor durch diese Lamellen hindurch nur den Teil des Körpers erfassen kann, der von dem Primärstrahl durchsetzt ist.

Wie durch die gestrichelte Linie 9 angedeutet, sind die Strahlenquelle 1, die Blenden 2 und 8 und die Detektoranordnung 5 mechanisch miteinander gekoppelt und senkrecht zum Primärstrahl 3 verschiebbar, so daß andere Bereiche im Körper 4 gemessen werden können. Es ist weiterhin möglich, diese Anordnung auch in einer zweiten zum Primärstrahl 3 senkrechten Richtung zu verschieben, so daß ein zweidimensionales Projektionsbild erstellt werden kann. Statt dessen kann die Anordnung jedoch auch um einen kleinen Winkel um eine zur Zeichenebene senkrechte Achse gedreht werden, wonach die Anordnung 1, 2, 5, 8 erneut verschoben wird - wie bei Computertomographen der 1. Generation üblich.

Die von den Detektoren gelieferten Meßwerte werden über einen Multiplexer 10 einem Analog-Digital-Wandler 11 mit linearer oder ggf. logarithmischer Kennlinie zugeführt und in einer Recheneinrichtung 12 weiterverarbeitet, die die Streusignale entsprechend der Schwächung des Primärstrahlenbündels korrigiert. Dabei wird von der Überlegung ausgegangen, daß die durch den Primärstrahl 3 erzeugte und unter einem geringen Winkel emittierte Streustrahlung im wesentlichen den gleichen Körperbereich durchsetzt und daher auch näherungsweise die gleiche Schwächung erfährt. Wenn beispielsweise im Zentrum eines Körpers ein Röntgenquant elastisch gestreut wird und das Maximum der Energie in einen Winkel von etwa 4^{o} mit dem Primärstrahl emittiert wird, dann haben die Austrittspunkte des Primärstrahls und des Streustrahls an der Peripherie des Körpers einen Abstand von nur 1 cm, so daß es gerechtfertigt ist, die Schwächung des Streustrahls und des Primärstrahls als gleich anzusetzen. Die Schwächung des Primärstrahls ist aber bekannt. Sie ergibt sich aus dem Verhältnis der Intensität der Primärstrahlung vor und hinter dem Körper, wobei die Intensität vor dem Eintritt in den Körper bekannt ist und die Intensität hinter dem Körper vom Detektor D₀ gemessen wird. Zur Korrektur der Streusignale müssen diese daher lediglich mit dem Schwächungsfaktor des Primärstrahls 3 multipliziert werden bzw. es muß (bei Logarithmierung der erhaltenen Meßwerte) zu dem erhaltenen Streusignal ein sich aus dem Schwächungsfaktor ergebender Betrag addiert werden.

Anschließend werden die so verarbeiteten Streusignale einem digitalen Speicher 13 zugeführt und in diesem nach Streuwinkeln getrennt gespeichert. Der Speicher 13 muß daher eine Speicherkapazität haben, die mindestens der Zahl der Projektionen multipliziert mit der Anzahl der Detektorelemente des Detektoranordnung 5 entspricht.

Aus dem Speicher 13 können die gespeicherten Werte wieder aufgerufen und zur Erzeugung eines Bildes benutzt werden.

Werden nur die Ausgangssignale des Detektors D₀ benutzt, so entspricht das rekonstruierte Bild einer üblichen Projektionsradiographie bzw. einem üblichen Computertomogramm. Werden hingegen nur die Ausgangssignale des Detektors D₅ - in der Recheneinheit 12 korrigiert mit Hilfe der Signale des Detektors D₀ - zur Erzeugung des Bildes benutzt, dann ergibt sich ein völlig abweichendes Bild und es werden vor allen Dingen diejenigen Stoffe dargestellt, die das Maximum der elastischen Streuung bei einem Winkel haben, der dem Winkel entspricht, den der Strahl 6 mit dem Strahl 3 einschließt. Werden hingegen die Signale eines anderen Detektors verarbeitet, ergibt sich wiederum ein anderes Bild.

Anstelle eines ganzen Bildes könnte aber auch die Information nur eines Teilbereiches des Bildes dargestellt werden. So könnte nach Erstellung eines Bildes, das aus den Ausgangssignalen des Detektors D₀ abgeleitet ist, das also die Absorptionsverteilung in den untersuchten Körperbereichen darstellt, vom Benutzer auf dem Monitor mit einem Lichtgriffel o.dgl. ein geeigneter Bereich markiert werden, dessen - über den markierten Bereich gemittelte - Streudichte als Funktion des Streuwinkels in einem Diagramm dargestellt wird.

Wenn der Körper aus amorphem Material besteht, verläuft die Streustrahlung kreissymmetrisch zum Primärstrahl. Dies macht es einerseits zulässig, zur Erfassung der Streustrahlung einen Detektor zu benutzen, der nur einen Teil der Streustrahlung erfassen kann, die unter einem bestimmten Winkel emittiert wird; andererseits erlaubt es, die Empfindlichkeit durch Verwendung von ringförmigen, zum Primärstrahl 3 konzentrischen Detektoren zu erhöhen.

Bei der Ausführungsform nach Fig. 1 wird durch die räumliche Anordnung der Detektoren 5, d.h. durch ihren relativ großen Abstand zum Körper 4, erreicht, daß jeder Detektor nur von Streustrahlung getroffen wird, die unter einem bestimmten Winkel zum Primärstrahl verläuft, der dem betreffenden Detektor fest zugeordnet ist. Es ist daher unmittelbar möglich, die den verschiedenen Stellungen des Röntgenstrahlers zugeordneten Werte nach Streuwinkeln getrennt zu speichern.

## Patentansprüche

1. Röntgengerät mit Strahlenquelle (1), die einen Primärstrahl (3) mit geringem Querschnitt emittiert und mit einer jenseits des Untersuchungsbereichs (4) angeordneten Detektoranordnung (5), die nur die im Primärstrahl (3) erzeugte, unter einem Winkel von weniger als 12^{o} zum Primärstrahl austretende Streustrahlung erfaßt, dadurch gekennzeichnet, daß die Strahlenquelle (1) und die Detektoranordnung miteinander gekoppelt und senkrecht zum Primärstrahl (3) verschiebbar sind, daß die Detektoranordnung (5) mehrere Detektoren (D₁..D₆) enthält, die solche Abmessungen haben und so weit vom Untersuchungsbereich (4) entfernt angeordnet sind, daß sie Streustrahlung jeweils nur unter einem bestimmten, dem betreffenden Detektor zugeordneten Streuwinkel von weniger als 12^{o} empfangen und entsprechende Streusignale liefern, daß die Detektoranordnung (5) zusätzlich einen im Primärstrahl angeordneten Detektor (D₀) zur Messung der Intensität des Primärstrahls hinter dem Untersuchungsbereich aufweist, daß eine Recheneinrichtung (12) vorgesehen ist, die Ausgangswerte bildet, die dem Produkt aus dem Streusignal und der durch den Quotienten aus der Intensität des Primärstrahls vor und hinter dem Untersuchungsbereich definierten Schwächung entsprechen, und daß ein Speicher (13) vorgesehen ist, in dem die den verschiedenen Stellungen der Strahlenquelle (1) zugeordneten Ausgangswerte nach Streuwinkeln getrennt gespeichert werden können.

2. Röntgengerät nach Anspruch 1, dadurch gekennzeichnet_{,} daß zwischen dem Untersuchungsbereich und der Detektoranordnung eine Blende (8) zur teilweisen Unterdrückung mehrfach gestreuter Strahlung vorgesehen ist.

## Claims

1. An X-ray apparatus, comprising a radiation source (1) which emits a primary beam (3) of small cross-section, and also comprising a detector array (5) which is arranged at the other side of the examination zone (4) and which detects only the scattered radiation generated within the primary beam (3) and emanating at an angle of less than 12° relative to the primary beam, characterized in that the radiation source (1) and the detector array are coupled to one another and are displaceable perpendicularly to the primary beam (3), that the detector array (5) comprises several detectors (D₁...D₆) which are so proportioned and situated at such a distance from the examination zone (4) that they detect scattered radiation only at a given scatter angle of less than 12° which is associated with the relevant detector, and supply corresponding scatter signals, that the detector array (5) also comprises a detector (D₀) which is arranged within the primary beam and measures the intensity of the primary beam behind the examination zone, that there is provided an arithmetic device (12) which forms output values which correspond to the product of the scatter signal and the attenuation defined by the quotient of the intensity of the primary beam in front of and behind the examination zone, and that there is provided a memory (13) in which the output values associated with the various positions of the radiation source (1) can be stored separately according to scatter angle.

2. An X-ray apparatus as claimed in Claim 1, characterized in that between the examination zone and the detector array there is provided a diaphragm (8) for the partial suppression of multiply scattered radiation.

## Revendications

1. Appareil à rayons X comportant une source de rayonnement (1) émettant un faisceau primaire (3) de section transversale faible, et un dispositif détecteur (5) disposé au-delà de la zone d'examen (4) et qui ne détecte que le rayonnement de diffusion engendré dans le faisceau primaire (3) et émergeant dans une direction faisant un angle de moins de 12° avec celle du faisceau primaire, caractérisé en ce que la source de rayonnement (1) et le dispositif détecteur sont couplés entre eux et peuvent être déplacés perpendiculairement au faisceau primaire (3), en ce que le dispositif détecteur (5) comporte plusieurs détecteurs (D₁ ...D₆) dont le dimensionnement et la distance par rapport à la zone d'examen (4) sont tels qu'ils ne reçoivent un rayonnement de diffusion que sous un angle de diffusion déterminé, attribué au détecteur concerné et inférieur à 12° et qu'ils fournissent des signaux de diffusion correspondants, en ce que le dispositif détecteur (5) présente en outre un détecteur (D₀) disposé dans le faisceau primaire et servant à mesurer l'intensité que présente le faisceau primaire derrière la zone d'examen, en ce qu'il est prévu un dispositif de calcul (12) formant des valeurs de sortie correspondant au produit du signal de diffusion et de l'atténuation définie par le quotient de l'intensité que présente le faisceau primaire devant et derrière la zone d'examen, et en ce qu'il est prévu une mémoire (13) dans laquelle peuvent être stockées, séparées selon les angles de diffusion, les valeurs de sortie attribuées aux différentes positions de la source de rayonnement (1).

2. Appareil à rayons X selon la revendication 1, caractérisé en ce qu'entre la zone d'examen et le dispositif détecteur, est prévu un obturateur (8) pour supprimer partiellement le rayonnement à diffusion multiple.
